# EUROPEAN PATENT APPLICATION

(11) **EP 4 549 550 A1**
(43) Date of publication of application: **07.05.2025**
(21) Application number: 23206758.7
(22) Date of filing: 30.10.2023
(51) Int. Cl.: C12N 5/071

(54) **LIVER SPHERE TRANSFER TO SCAFFOLDS**

(71) Applicant: Stimuliver ApS, 2200 København N (DK)
(72) Inventor: SZKOLNICKA, Dagmara, 2200 København N (DK); GONZÁLEZ DEL BARRIO, Lydia, 2200 København N (DK); QUINTANA CALDERÓN, Carlos, 2200 København N (DK); C. HAY, David, 2200 København N (DK)
(74) Representative: Zacco Denmark A/S

(57) **Abstract**

The present invention relates to methods for preparing liver tissue and the liver tissue obtained by the method. The present invention further relates to the therapeutic use of the liver tissue obtained by the method.

## Description

### Field of the invention

The present invention relates to methods for preparing liver tissue and the liver tissue obtained by the method. The present invention further relates to the therapeutic use of the liver tissue obtained by the method.

### Background of the invention

Stem cell-based tissue engineering applications aim to capture key features of development, for example to promote *ex vivo* tissue formation for the replacement of malfunctioning/defective or injured tissues. Stem cells, in particular pluripotent stem cells, are useful for such applications due to their capacity to self-renew and differentiate into a plurality of differentiated cell types.

Pluripotent stem cell-derived liver tissue represents a potential alternative to cadaver derived liver tissue. Simple and efficient, two-dimensional (2D) differentiation procedures have been developed, with commercial kits available. Although facile, 2D systems deliver liver tissue lacking certain functions and display significant phenotypic instability. Efforts to overcome these limiting factors have led to the building of three-dimensional (3D) cellular aggregates. Although those systems, display more stable phenotype, their widespread application and adoption is limited due to the reliance on poorly defined biological components. Part of the issue associated with this type of tissue engineering approach is the spontaneous nature of cellular differentiation, making it susceptible to variable cell specification and phenotype. To address this, we have developed an economic and automated platform to produce human liver tissue that precisely controls cells input and cell potency. In combination with microwell driven tissue engineering and scaffolds our system performs consistently within and between experiments. There is a need in the art for improved methods for producing and preparing liver tissue suitable for therapeutic application.

### Summary of the invention

The object of the present invention relates to the provision of improved methods for preparing liver tissue, in particular liver tissue on a scaffold.

In a first aspect, the present invention provides a method for preparing liver tissue, said method comprising the step of:
(i) providing endothelial cells;
(ii) providing hepatic progenitor cells;
(iii) preparing a mixture comprising said hepatic progenitor cells and endothelial cells in a culture medium;
(iv) seeding the mixture on a cell culture substrate;
(v) allowing the mixture of cells to propagate and form an aggregation of cells;
(vi) collecting said aggregation of cells such as after about 1 to 6 days;
(vii) seeding said aggregation of cells on a scaffold and allowing the mixture of cells to propagate and form liver tissue on said scaffold.

In a second aspect, the present invention provides liver tissue obtained by the method of the present invention.

In a further aspect, the present invention provides said liver tissue for use as a medicament such as for use in the treatment of acute-on-chronic liver failure (ACLF), acute liver failure, chronic liver failure, preparation for liver implantation, tyrosinemia type I or Alpha-1 antitrypsin deficiency (A1AD or AATD).

### Brief description of the drawings

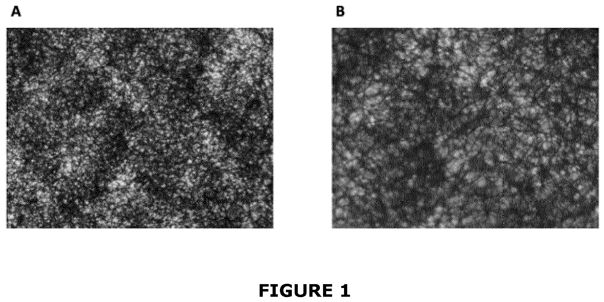

**Figure 1****:** Optical microscope images of the empty polycaprolactone scaffold of 0.5 mm thickness and 8 µm fiber diameter. Images obtained through EVOS^{™} light microscopy at 4x (A) and 10x magnification (B).

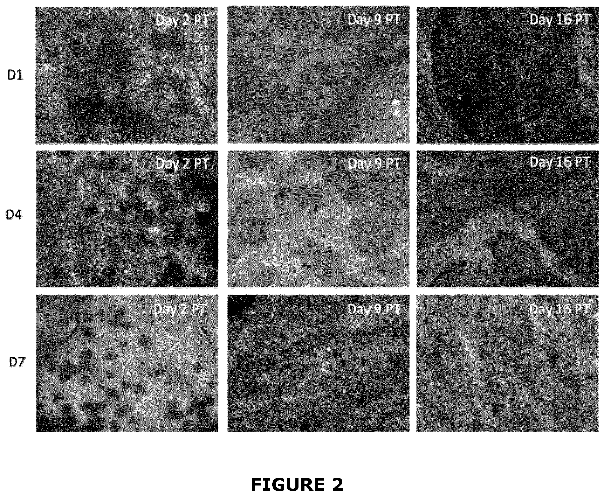

**Figure 2****:** Optical microscope images of the polycaprolactone scaffold of 0.5 mm thickness and 8 µm fiber diameter with liver spheres. Each row represents scaffolds with liver spheres that were transferred 1 day after cell aggregation (D1), 4 days after cell aggregation (D4) or 7 days after cell aggregation (D7). Each column displays representative images of the liver spheres at different days after they were transferred to the scaffold. PT signifies "post transfer". Images obtained through EVOS^{™} light microscopy at 4x magnification.

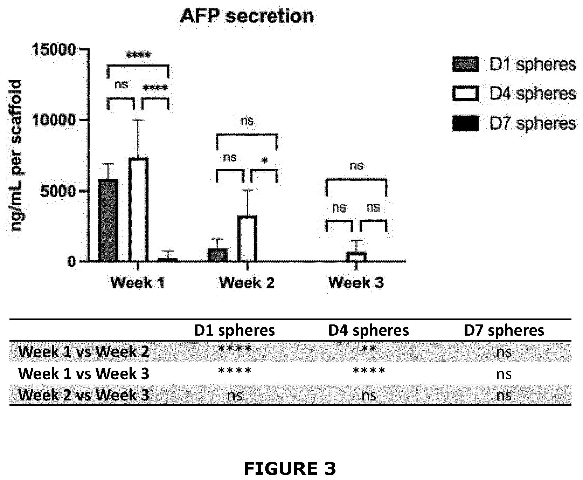

**Figure 3****:** Graph describing secretion Alpha fetoprotein (AFP) per scaffold over a period of 3 weeks. Data are expressed as mean + SD of 4 independent biological replicates. Two-way ANOVA was performed for each week comparing D1 vs D4 vs D7 (graph) and for D1, D4 and D7 of specific weeks (table). P values legend: ns P > 0.05; * P < 0.0332; ** P < 0.0021; *** P < 0.0002; *** P < 0.0001. Data were analyzed by Graphpad (Prism 9).

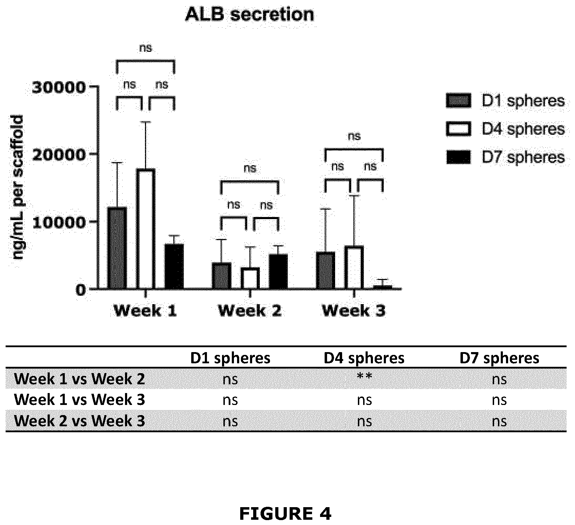

**Figure 4****:** Graph describing Albumin (ALB) per scaffold over a period of 3 weeks. Data are expressed as mean + SD of 4 independent biological replicates. Two-way ANOVA was performed for each week comparing D1 vs D4 vs D7 (graph) and for D1, D4 and D7 of specific weeks (table). P values legend: ns P > 0.05; * P < 0.0332; ** P < 0.0021; *** P < 0.0002; *** P < 0.0001. Data were analyzed by Graphpad (Prism 9).

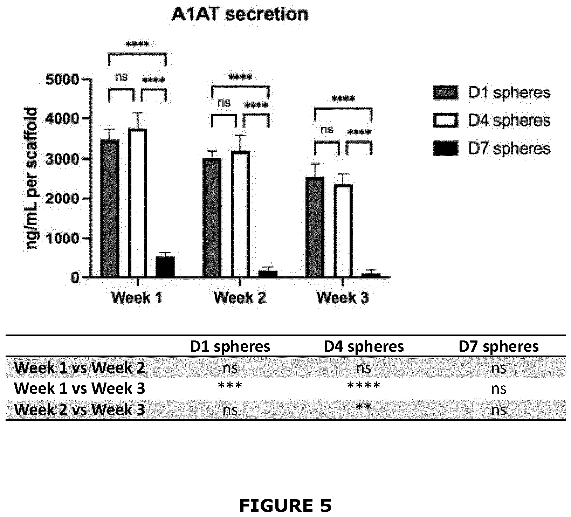

**Figure 5****:** Graph describing secretion of Alpha1-antitrypsin (A1AT) per scaffold over a period of 3 weeks. Data are expressed as mean + SD of 4 independent biological replicates. Two-way ANOVA was performed for each week comparing D1 vs D4 vs D7 (graph) and for D1, D4 and D7 of specific weeks (table). P values legend: ns P > 0.05; * P < 0.0332; ** P < 0.0021; *** P < 0.0002; *** P < 0.0001. Data were analyzed by Graphpad (Prism 9).

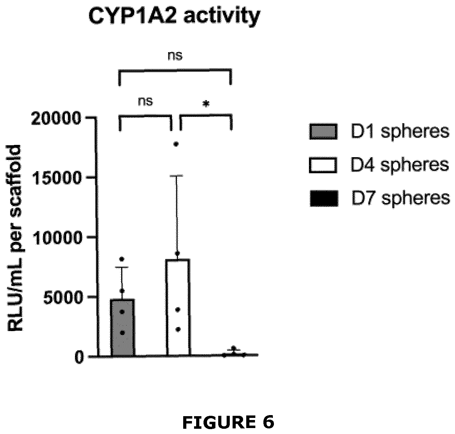

**Figure 6****:** Graph describing activity of Cytochrome P450 1A2 (CYP1A2) per scaffold at 3 weeks. Data are expressed as mean + SD of 4 independent biological replicates. Kruskal-Wallis H test was performed comparing D1 vs D4 vs D7. P values legend: ns P > 0.05; * P < 0.0332; ** P < 0.0021; *** P < 0.0002; *** P < 0.0001. Data were analyzed by Graphpad (Prism 9). RLU = Relative Light Units.

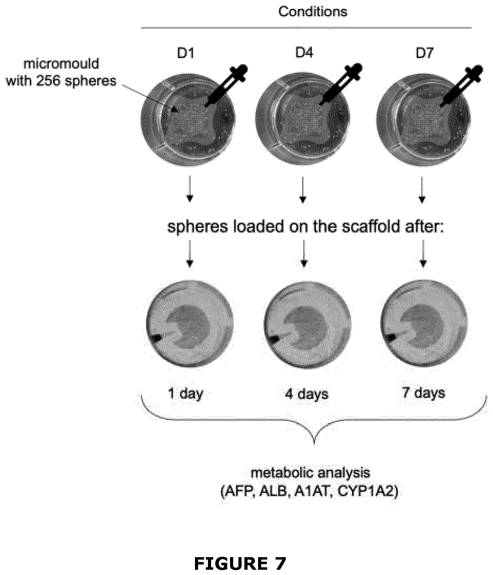

**Figure 7****:** Stem cell derived liver spheres were generated in the micromoulds and were subsequently transferred either 1 day (D1), 4 days (D4) or 7 days (D7) after aggregation to the polycaprolactone scaffold (8um fibre diameter; 0.5 mm in depth). Post-transferred spheres were subjected for metabolic activity examination for 1-3 weeks.

### Detailed description of the invention

The present invention relates to methods for preparing liver tissue and the liver tissue obtained by the method. The present invention further relates to the therapeutic use of the liver tissue obtained by the method.

A more complete understanding of methods of the present invention can be obtained by reference to the detailed description of the invention and by reference to the examples and accompanying drawings. The examples and drawings represent working examples and are therefore not intended to define or limit the scope of the exemplary embodiments.

A stem cell is an undifferentiated cell from which specialized cells are subsequently derived. Pluripotent stem cells can be differentiated into any of the three germ layers: endoderm, mesoderm, or ectoderm. Post fertilization, pluripotent stem cells form every cell type in the human body, including less plastic stem cell populations such as adult stem cells, fetal stem cells, and amniotic stem cells.

Embryonic stem cells are a type of pluripotent stem cell, and possess extensive self-renewal capacity and pluripotency. Another type of pluripotent stem cell, induced pluripotent stem cells, were produced from mammalian terminally differentiated cells by a process termed somatic cell reprogramming. The process by which a stem cell changes into a more specialized cell is referred to as differentiation, for example the differentiation of endodermal progenitor cells to hepatocytes.

The present invention provides methods for preparing liver tissue and the liver tissue obtained by the method of the present invention.

Accordingly in one aspect, the present invention provides a method for preparing liver tissue, said method comprising the step of:
(i) providing endothelial cells;
(ii) providing hepatic progenitor cells;
(iii) preparing a mixture comprising said hepatic progenitor cells and endothelial cells in a culture medium;
(iv) seeding the mixture on a cell culture substrate;
(v) allowing the mixture of cells to propagate and form an aggregation of cells;
(vi) collecting said aggregation of cells such as after about 1 to 6 days;
(vii) seeding said aggregation of cells on a scaffold and allowing the mixture of cells to propagate and form liver tissue on said scaffold.

In one embodiment, the mixture of cells is allowed to propagate and form an aggregation of cells before collecting the aggregation of cells after 1 to 7 days, such as after 1 to 6 days, such as after 1 to 5 days, for example 1 to 4 days, for example 2 to 4 days, such as about 4 days. In another embodiment, the aggregation of cells is collected after 1, 2, 3, 4, 5, 6 or 7 days.

In one embodiment, the mixture of cells comprises hepatic progenitor cells and endothelial cells. In another embodiment, the mixture of cells consists or consists essentially of hepatic progenitor cells and endothelial cells. In a further embodiment, the ratio of endothelial cells to hepatic progenitor cells in the mixture is in the range of 1:1 to 1:3.5, for example 1:1 to 1:3, such as 1:1.5, 1:2 or 1:3.3.

The present invention further relates to the therapeutic use of the liver tissue obtained by the method.

The method of the present invention uses a mixture of hepatic progenitor cells and endothelial cells. Hepatic progenitor cells (also known as hepatoblasts) is a fetal precursor of the hepatocyte. In particular, hepatoblasts are parenchymal cells of fetal livers and are capable of self-renewal in culture and differentiation into hepatocytes or biliary epithelium.

The hepatic progenitor cells and/or endothelial cells are typically obtained by differentiation of pluripotent stem cells, such as human pluripotent stem cells. In one embodiment, pluripotent stem cells are selected from embryonic stem cells, induced pluripotent stem cells, adult stem cells, fetal stem cells, amniotic stem cells, and any combination thereof. In one embodiment, the pluripotent stem cells are mammalian pluripotent stem cells. In a preferred embodiment, the pluripotent stem cells are human pluripotent stem cells. In a further embodiment, the pluripotent stem cells are human pluripotent stem cells, which have not been obtained by destruction of human embryos. Human pluripotent stem cells may express one or more of the markers (or all markers) from the list consisting of SSEA-3, SSEA- 4, TRA-1-60, TRA-1-81, TRA-2-49/6E, ALP, Sox 2, E-cadherin, UTF-1, Oct4, Lin28, Rex-1, and Nanog.

In *vitro*/*ex vitro* differentiation of stem cells generally includes a substrate or coating that provides a structural support for the cell; and a cell culture medium to provide nutrition to the cell. The substrate or coating is typically formed as a layer in a container, for example a petri dish, in the well of a multi-well plate or flasks such as T flasks, for example T75 or T175 flasks.

In one embodiment of the present invention, the endothelial cells used by the method have been obtained by differentiating pluripotent stem cells, such as human pluripotent stem cells to endothelial cells on a cell culture substrate comprising the combination of at least two laminins, i.e. laminin-521 and laminin-111 (or a functional fragment thereof or one or both of the laminins).

In another embodiment of the present invention, the hepatic progenitor cells used by the method have been obtained by differentiating pluripotent stem cells, such as human pluripotent stem cells to endothelial cells on a cell culture substrate comprising the combination of at least two laminins, i.e. laminin-521 and laminin-111 (or a functional fragment thereof or one or both of the laminins).

In yet a further embodiment, the hepatic progenitor cells and the endothelial cells used by the method have been obtained by differentiating pluripotent stem cells, such as human pluripotent stem cells to endothelial cells on a cell culture substrate comprising the combination of at least two laminins, i.e. laminin-521 and laminin-111 (or a functional fragment thereof or one or both of the laminins).

Laminins are a family of heterotrimeric glycoproteins that reside primarily in the basal lamina. They function via binding interactions with neighboring cell receptors on the one side, and by binding to other laminin molecules or other matrix proteins such as collagens, nidogens or proteoglycans. The laminin molecules are also important signaling molecules that can strongly influence cellular behavior and function. Laminins are important in both maintaining cell/tissue phenotype, as well as in promoting cell growth and differentiation in tissue repair and development.

A laminin protein molecule comprises one alpha-chain subunit, one beta-chain subunit, and one gamma-chain subunit, all joined together in a trimer through a coiled-coil domain. There exist five different alpha chains, three beta chains and three gamma chains that in human tissues have been found in at least fifteen different combinations.

In the context of the present invention the term "laminin-521" refers to the protein formed by joining alpha5, beta2 and gamma1 chains together. The term laminin-111 (laminin-1) refers to the polypeptide that contains alpha-1, beta-1 and gamma-1 chains.

In the context of the present invention, reference is made to fragments of laminin-521 and fragments of laminin-111. It should be understood that the recited fragments comprise the domains of the full polypeptide that is essential for the maintaining an activity corresponding essentially to that of the full-length laminin.

The laminin may be obtained by recombinant expression in any suitable host cell. Such laminin is referred to as a recombinant laminin. Alternatively, the laminin from natural sources (non-recombinant).

In order to comply with good manufacturing practice (GMP), the laminins used in the preparation of the hepatic progenitor cells and the endothelial cells are isolated laminins, for example laminins obtained by recombinant expression in a suitable host.

Basement membrane extracts such as Matrigel, Cultrex and Geltrex comprise laminins. However, these products are commonly occurring lot-to-lot variability during manufacturing and complexity in composition, which are often poorly defined, making it difficult to determine exactly which signals are promoting cell function. Thus, the use of Matrigel, Cultrex and Geltrex (animal-derived matrix) does not comply with good manufacturing practice (GMP) and therefore none of these products is used in the method of the present invention. In one embodiment of the present invention, the methods of the invention compatible with good manufacturing practice (GMP). Thus, the cells used by and tissue obtained by the method are compatible with good manufacturing practice (GMP) and thus void of unknown factors that render the cell and tissues incompatible with subsequent clinical applications.

In a preferred embodiment, the laminins used in the preparation of the hepatic progenitor cells and the endothelial cells are human laminins, such as human laminin 521 and human laminin 111. In another preferred embodiment, the laminins including human laminin 521 and human laminin 111 are obtained by recombinant expression. A laminin obtained by recombinant expression is referred to as a recombinant laminin. The cell culture substrate on which the pluripotent stem cells are seeded may be a matrix consisting of or essentially consisting of laminin 521 and laminin 111 (or functional fragments thereof), for example human laminin 521 and human laminin 111. In a further embodiment, the cell culture substrate comprises laminin 521 and laminin 111 (or functional fragments thereof), such as recombinant human laminin 521 and human laminin 111. In a further embodiment, the cell culture substrate comprises laminin 521 and laminin 111 and one or more additional components, for example matrix proteins, such as collagen I and fibronectin.

Preferably, the polypeptide components of the cell culture substrate including laminin 521 and laminin 111 are recombinant polypeptides. In the context of the present invention, the term "recombinant" polypeptide refers to a polypeptide, which is produced by expression from an exogeneous polynucleotide encoding the polypeptide in a suitable host cell.

In one embodiment, the hepatic progenitor cells and/or the endothelial cells used by the method of the present invention is obtained by seeding pluripotent stem cells on a cell culture substrate comprising, consisting of or essentially consisting of laminin-521 (or a fragment of laminin-521) and laminin-111 (or a fragment of laminin-111) and differentiating the cells to obtain hepatic progenitor cells and/or the endothelial cells by contacting the cells with differentiation factors the induce the desired differentiation. Preferably, the laminin-521 and laminin-111 are human laminins such as recombinant human laminins.

In one embodiment, the concentration of laminin-521 and laminin-111 in said cell culture substrate is in the range of about 1 to 10 microgram(s)/ml, for example 3 to 8 microgram(s)/ml, such as 4 to 7 microgram(s)/ml, for example 5 to 6 microgram(s)/ml, such as about 5 ug/ml, such as 1.25 microgram(s) laminin-521/ml and 3.75 microgram(s) laminin-111/ml.

The above amount of laminin may also be expressed as microgram(s)/cm². Thus, in one embodiment, the concentration of laminin-521 and laminin-111 on said cell culture substrate is 1 microgram(s)/10cm² - 10 microgram(s)/10cm², for example 3 microgram(s)/10cm² - 8 microgram(s)/10cm², such as 4 microgram(s)/10cm² - 7 microgram(s)/10cm², for example 5 to 6 microgram(s)/10cm², such as about 5 microgram(s)/10cm², such as 1.25 microgram(s) laminin-521/10cm² and 3.75 microgram(s) laminin-111/10cm².

In another embodiment, the concentration by weight of laminin-111 in said cell culture substrate is equal to or higher than the concentration of laminin-521, such as the ratio of laminin-111 to laminin-521 is in the range of 1:1 to 4:1, such as about 3:1. In one embodiment, the ratio of laminin-111 to laminin-521 (by weight) is about 3:1.

The pluripotent stem cells are typically seeded on said culture substrate at a density of 25000 to 40000 pluripotent stem cells per cm², such as 30000 to 35000 pluripotent stem cells per cm², for example 30000 pluripotent stem cells per cm². The inventors have discovered that the optimal seeding density of the pluripotent stem cells is about 30000 pluripotent stem cells per cm². Thus, in one embodiment, the pluripotent stem cells used for differentiating endothelial cells are seeded at a density of about 30000 pluripotent stem cells per cm². In another embodiment, the pluripotent stem cells used for differentiating hepatic progenitors are seeded at a density of about 40000 pluripotent stem cells per cm².

After the pluripotent stem cells have been seeded on the culture substrate, the cells are allowed to expand by proliferation to reach a confluency in the range of about 30 to 60%, such as 30 to 55% such as 30 to 50%, for example 40 to 50%, such as about 40% before contacting the cells with differentiation factors. Thus in one embodiment, said one or more differentiation factors is added when the cells have reached a confluency in the range of about 30 to 60%, such as 30 to 55% such as 30 to 50%, for example 40 to 50%, such as about 40%. In another embodiment, said one or more differentiation factors is added when the cells have reached a confluency about 40%.

The hepatic progenitor cells used by the method of the present invention have typically been obtained by differentiation of the pluripotent stem cells, such as human pluripotent stem cells, to hepatic progenitor cells.

The differentiation of the pluripotent stem cells to hepatic progenitor cells is characterized by markers, which may be used to identify and isolate the hepatic progenitor cells from a population of cells comprising hepatic progenitor cells and cells, which are not differentiated. Immunocytochemistry, flow cytometry, and enzymatic analysis and RT-PCR are examples of methods for analysis of the cell population. MACS or FACS sorting are examples of methods that may be used to isolate hepatic progenitor cells from the population of cells.

In one embodiment, the hepatic progenitor cells used by the method have been enriched for Alpha-fetoprotein (AFP) and HNF4a positive cells. In a further embodiment, 90 to 100% of the hepatic progenitor cells are positive of AFP and/or HNF4a. The amount of AFP and HNF4a positive cells may be determined using methods known in the art, such as immunostaining (HNF4a) and ELISA (AFP).

In another embodiment, 90-100% of the hepatic progenitor cells are positive for AFP and 90-100% of the hepatic progenitor cells are positive for HNF4a, such as 95-100% positive for AFP and 95-100% positive for HNF4a. In the process of preparing the liver tissue the hepatic progenitor cells will mature and the AFP marker (foetal marker) will decrease, whereas markers for maturation will appear such that the hepatocytes in the aggregation typically will be positive for Albumin, HNF4a, CYP1A2 and A1AT. In one embodiment, the liver cells of the liver tissue produced by the method are positive for HNF4a and display CYP1A2 function and albumin and A1AT secretion. The positive cells may be determined using methods known in the art, such as immunostaining (HNF4a, Albumin) and ELISA (AFP, A1AT).

In one embodiment, the hepatic progenitor cells used by the method of the present invention have been obtained by differentiating pluripotent stem cells, preferably human pluripotent stem cells, to hepatic progenitor cells by culturing the pluripotent stem cells on a cell culture substrate comprising laminin 521 and laminin 111 (or functional fragments of thereof) and contacting the cells with differentiation factors that induce the hepatic differentiation.

In another embodiment, the hepatic progenitor cells have been obtained by seeding pluripotent stem cells on a cell culture substrate comprising a mixture of laminin-521 or a fragment thereof and laminin-111 or a fragment thereof; and culturing the pluripotent stem cells to obtain said hepatic progenitor cells. In one embodiment, the weight ratio of laminin-111 to laminin-521 is from about 4:1 to about 1:1.

In one embodiment, the hepatic progenitor cells used by the method of the present invention have been obtained by culturing the pluripotent stem cells in the presence of activin A and Wnt3a. In a further embodiment, the hepatic progenitor cells have been obtained by culturing the pluripotent stem cells in the presence of activin A and Wnt3a followed by culturing the pluripotent stem cells in the presence of hydrocortisone (HC), hepatocyte growth factor (HGF), and oncostatin m (OSM).

The endothelial cells used by the method of the present invention have typically been obtained by differentiation of the pluripotent stem cells, such as human pluripotent stem cells, to endothelial cells.

The differentiation of the pluripotent stem cells to endothelial cells is characterized by markers, which may be used to identify and isolate the endothelial cells from a population of cells comprising endothelial cells and cells, which are not differentiated. Immunocytochemistry, flow cytometry, and enzymatic analysis and RT-PCR are examples of methods for analysis of the cell population. MACS or FACS sorting are examples of methods that may be used to isolate endothelial cells from the population of cells.

Markers for endothelial cells includes VE-cadherin (VE-CADH, CD144) and cluster of differentiation 31 (CD31).

In one embodiment, about 0% of the endothelial cells used by the method of the present invention are negative for Alpha-fetoprotein (AFP) markers. In another embodiment, the endothelial cells are positive for at least one marker selected from the list consisting of: VE-cadherin (VE-CADH, CD144) positive, CD31 positive and the combination of VE-cadherin (VE-CADH, CD144) and CD31 positive.

In one embodiment, the endothelial cells have been obtained by isolating VE-cadherin (VE-CADH, CD144) positive and/or CD31 positive cells, such as by MACS or FACS sorting, such as by isolating VE-cadherin (VE-CADH, CD144) positive cells.

In one embodiment, the endothelial cells used by the method of the present invention have been enriched for VE-cadherin (VE-CADH, CD144) positive cells. In another embodiment, at least 20% of the endothelial cells used by the method are VE-cadherin (VE-CADH, CD144) positive endothelial cells, such as at least 30% VE-cadherin (VE-CADH, CD144) positive endothelial cells, for example at least 40% VE-cadherin (VE-CADH, CD144) positive endothelial cells, such as at least 50% VE-cadherin (VE-CADH, CD144) positive endothelial cells, for example at least 60% VE-cadherin (VE-CADH, CD144) positive endothelial cells, such as at least 70% VE-cadherin (VE-CADH, CD144) positive endothelial cells, such as 20 to 40% VE-cadherin (VE-CADH, CD144) positive endothelial cells, for example 20 to 70% VE-cadherin (VE-CADH, CD144) positive endothelial cells, such as 40 to 70% VE-cadherin (VE-CADH, CD144) positive endothelial cells, for example 50 to 70% VE-cadherin (VE-CADH, CD144) positive endothelial cells, such as 60 to 70% VE-cadherin (VE-CADH, CD144) positive endothelial cells.

Endothelial cells are also characterized by being negative for the Alpha-fetoprotein (AFP) marker.

In one embodiment, the endothelial cells used by the method of the present invention cluster of differentiation 31 (CD31) positive endothelial cells. In one embodiment, at least 20% of the endothelial cells used by the method are cluster of differentiation 31 (CD31) positive endothelial cells, such as at least 30% cluster of differentiation 31 (CD31) positive endothelial cells, for example at least 40% cluster of differentiation 31 (CD31) positive endothelial cells, such as at least 50% cluster of differentiation 31 (CD31) positive endothelial cells, for example at least 60% cluster of differentiation 31 (CD31) positive endothelial cells, such as at least 70% cluster of differentiation 31 (CD31) positive endothelial cells, for example at least 80% cluster of differentiation 31 (CD31) positive endothelial cells, such as 20 to 80% cluster of differentiation 31 (CD31) positive endothelial cells.

In one embodiment, the endothelial cells used by the method of the present invention have been obtained by differentiating pluripotent stem cells, preferably human pluripotent stem cells, to endothelial cells by culturing the pluripotent stem cells on a cell culture substrate comprising a mixture laminin 521 and laminin 111 (or functional fragments of thereof) and contacting the cells with differentiation factors that induce the endothelial differentiation.

In another embodiment, the endothelial cells have been obtained by seeding pluripotent stem cells on a cell culture substrate comprising a mixture of laminin-521 or a fragment thereof and laminin-111 or a fragment thereof, wherein the concentration by weight of laminin-111 in said cell culture substrate is equal to or higher than the concentration of laminin-521, such as the ratio of laminin-111 to laminin-521 is in the range of 1:1 to 4:1, such as about 3:1.

In one embodiment, the endothelial cells have been obtained by culturing the pluripotent stem cells in the presence of CHIR99021 (CHIR) and Bone morphogenetic protein 4 (BMP4).

CHIR99021 (CHIR) and/or Bone morphogenetic protein 4 (BMP4) is typically used to stimulate the differentiation of pluripotent stem cells to mesodermal cells and in a subsequent step, differentiation factors (such as VEGF and/or forskolin) are introduced to induce differentiation to endothelial cells.

Accordingly, in one embodiment, the endothelial cells have been obtained by culturing the pluripotent stem cells in the presence of CHIR99021 (CHIR) and Bone morphogenetic protein 4 (BMP4) followed by culturing the pluripotent stem cells in the presence of Vascular endothelial growth factor (VEGF) and forskolin.

In one embodiment, the hepatic progenitor cells are human hepatic progenitor cells. In another embodiment, the endothelial cells are human endothelial cells. In a further embodiment, the hepatic progenitor cells and the endothelial cells are human cells. In yet another embodiment, the said liver tissue produced by the method is human liver tissue. In one embodiment, 90 to 100% of the liver cells are positive for HNF4a and 90 to 100% display CYP1A2 function and 90 to 100% are positive for albumin and 90 to 100% are positive for A1AT secretion.

The method of the present invention comprises seeding the mixture comprising said hepatic progenitor cells and endothelial cells in a culture medium on a cell culture substrate, typically in the form of a scaffold.

The selection of tissue engineering scaffold material is directly associated with the effect of the material implanted in the animal or human body. Most importantly, the scaffold materials should have good biocompatibility. Scaffold materials can provide a good adsorption interface for the adhesion of seed cells, in order to facilitate cell proliferation. In addition, the scaffold material must have a certain level of biomechanical strength and maintain a controlled degradation rate once implanted into the animal or human body. The material should have no cytotoxic, immunogenic, tumorigenic, or teratogenic effects on animals or humans. Scaffold materials include natural biological scaffold materials, synthetic biodegradable polymer scaffold materials, composite scaffold materials.

The scaffold used by the method of the present invention typically comprises or consist of one or more biodegradable scaffolds materials, for example biodegradable polymers, such as synthetic biodegradable polymer scaffold materials. In one embodiment, the scaffold comprises a polymer, such as a biodegradable polymer. In one embodiment, the scaffold comprises polysaccharides and/or polypeptides. In another embodiment, the scaffold is selected from the group consisting of fibrous scaffold, porous scaffold and hydrogel scaffold.

In one embodiment, the scaffold comprises a polymer selected from the group consisting of polycaprolactone (PCL), polyacrylate-coated polycaprolactone, and polyurethane. In another embodiment, the scaffold comprises, consists or consists essentially of polycaprolactone (PCL).

In one embodiment, the scaffold comprises, consists or consists essentially of polycaprolactone (PCL) fibers having a diameter 1 to 10 micrometer(s), such as 4 to 8 micrometer(s), for example about 8 micrometers.

In one embodiment, the depth of the scaffold is in the range of 0.2 to 2 millimeters, such as 0.5 to 1 millimeters. The depth of the scaffold defines the cavity of the scaffold, where the mixture of cells is seeded.

In one embodiment, the said scaffold has a surface in the range of 0.5 to 1.5 cm², for example 0.7 to 1.25 cm² , such as 0.7 to 1 cm², for example 0.7 to 0.95 cm², such as example 0.7 to 0.85 cm², or for example about 0.79 or 0.95 cm². For example, the method is performed in 24 well trays where the scaffold in each well typically has an area of 0.7 to 0.95 cm², about 0.95 cm²

In a further aspect, the present invention provides the liver tissue obtained by the method of the present invention. In one embodiment, the 90 to 100% of the liver cells are positive for HNF4a and 90 to 100% display CYP1A2 function and 90 to 100% are positive for albumin and 90 to 100% are positive for A1AT secretion. The presence of the markers may be determined using methods of the prior arts described herein.

In a further embodiment, the liver cells do not or essentially do not express fetal liver protein (AFP). For example, at three weeks from the preparation of the liver tissue the amount of fetal liver protein (AFP) is typically less than 2000 ng/mL, for example less than 1000 ng/mL.

In a further aspect of the present invention, the liver tissue obtained by the method of the present invention is for use as a medicament.

In some embodiment, the liver tissue obtained by the method of the present invention is for use in the treatment of acute liver failure, chronic liver failure, preparation for liver implantation, tyrosinemia type I or Alpha-1 antitrypsin deficiency (A1AD or AATD). In one embodiment, the liver tissue is for use in the treatment of acute-on-chronic liver failure (ACLF), acute liver failure, chronic liver failure, or preparation for liver implantation.

The present invention further provides a method for treating, aveliating, reducing, slowing down and/or preventing acute liver failure, chronic liver failure, prepatation for liver implantation, tyrosinemia type I or Alpha-1 antitrypsin deficiency (A1AD or AATD) in a subject, said method comprising administrating a therapeutic effective amount of the liver tissue obtained by the method of the present invention.

The present invention is further characterized in the following non-limiting embodiments:
Embodiment 1. A method for preparing liver tissue, said method comprising the step of:
   (i) providing endothelial cells;
   (ii) providing hepatic progenitor cells;
   (iii) preparing a mixture comprising said hepatic progenitor cells and endothelial cells in a culture medium;
   (iv) seeding the mixture on a cell culture substrate;
   (v) allowing the mixture of cells to propagate and form an aggregation of cells;
   (vi) collecting said aggregation of cells such as after about 1 to 6 days;
   (vii) seeding said aggregation of cells on a scaffold and allowing the mixture of cells to propagate and form liver tissue on said scaffold.
Embodiment 2. The method according to embodiment 1, wherein the aggregation is collected after 1, 2, 3, 4, 5, 6 or 7 days.
Embodiment 3. The method according to embodiment 1 or 2, wherein the aggregation is collected after 1 to 4 days, such as about 1 day or about 4 days.
Embodiment 4. The method according to any one of the preceding embodiments, wherein the ratio of said endothelial cells to hepatic progenitor cells in the mixture is in the range of 1:1 to 1:3.5, for example 1:1 to 1:3, such as 1:1.5, 1:2 or 1:3.3.
Embodiment 5. The method according to any one of the preceding embodiments, wherein the endothelial cells have been enriched for CD144 positive cells.
Embodiment 6. The method according to any one of the preceding embodiments, wherein at least 20% of the endothelial cells are CD144 positive, such as at least 25% of the endothelial cells are CD144 positive, for example at least 30% of the endothelial cells are CD144 positive, such as 20 to 40% of the endothelial cells CD144 positive.
Embodiment 7. The method according to any one of the preceding embodiments, wherein the hepatic progenitor cells have been enriched for AFP and HNF4a positive cells.
Embodiment 8. The method according to any one of the preceding embodiments, wherein 90 to 100% of the hepatic progenitor cells are positive of AFP and/or HNF4a.
Embodiment 9. The method according to any one of the preceding embodiments, wherein said hepatic progenitor cells have been obtained by seeding pluripotent stem cells on a cell culture substrate comprising a mixture of laminin-521 or a fragment thereof and laminin-111 or a fragment thereof; and culturing the pluripotent stem cells to obtain said hepatic progenitor cells.
Embodiment 10. The method according to embodiment 9, wherein the weight ratio of laminin-111 to laminin-521 is from about 4:1 to about 1:1.
Embodiment 11. The method according to any one of the preceding embodiments 9 and 10, wherein said hepatic progenitor cells have been obtained by culturing the pluripotent stem cells in the presence of activin A and Wnt3a.
Embodiment 12. The method according to embodiment 11, wherein said hepatic progenitor cells have been obtained by culturing the pluripotent stem cells in the presence of activin A and Wnt3a followed by culturing the pluripotent stem cells in the presence of hydrocortisone (HC), hepatocyte growth factor (HGF), and oncostatin m (OSM).
Embodiment 13. The method according to any one of the preceding embodiments, wherein said endothelial cells have been obtained by seeding pluripotent stem cells on a cell culture substrate comprising a mixture of laminin-521 or a fragment thereof and laminin-111 or a fragment thereof; and culturing the pluripotent stem cells to obtain said endothelial cells.
Embodiment 14. The method according to embodiment 13, wherein said endothelial cells have been obtained by seeding pluripotent stem cells on a cell culture substrate comprising a mixture of laminin-521 or a fragment thereof and laminin-111 or a fragment thereof, wherein the concentration by weight of laminin-111 in said cell culture substrate is equal to or higher than the concentration of laminin-521, such as the ratio of laminin-111 to laminin-521 is in the range of 1:1 to 4:1, such as about 3: 1.
Embodiment 15. The method according to any one of the preceding embodiments 13 and 14, wherein said endothelial cells have been obtained by culturing the pluripotent stem cells in the presence of CHIR99021 (CHIR) and Bone morphogenetic protein 4 (BMP4).
Embodiment 16. The method according to any one of the preceding embodiments 13 and 15, wherein said endothelial cells have been obtained by culturing the pluripotent stem cells in the presence of CHIR99021 (CHIR) and Bone morphogenetic protein 4 (BMP4) followed by culturing the pluripotent stem cells in the presence of Vascular endothelial growth factor (VEGF) and forskolin.
Embodiment 17. The method according to any of the preceding embodiments, wherein said pluripotent stem cells are selected from embryonic stem cells, induced pluripotent stem cells, adult stem cells, fetal stem cells, amniotic stem cells, and any combination thereof.
Embodiment 18. The method according to any of the preceding embodiments, wherein said pluripotent stem cells are human pluripotent stems cells.
Embodiment 19. The method according to any of the preceding embodiments, wherein said liver tissue is human liver tissue.
Embodiment 20. The method according to any of the preceding embodiments, wherein said hepatic progenitor cells are human hepatic progenitor cells.
Embodiment 21. The method according to any of the preceding embodiments, wherein said endothelial cells are human endothelial cells.
Embodiment 22. The method according to any one of embodiments 1 to 21, wherein 90 to 100% of the liver cells are positive for HNF4a and 90 to 100% display CYP1A2 function and 90 to 100% are positive for albumin and 90 to 100% are positive for A1AT secretion.
Embodiment 23. The method according to any of the preceding embodiments, wherein said scaffold is selected from the group consisting of fibrous scaffold, porous scaffold and hydrogel scaffold.
Embodiment 24. The method according to any of the preceding embodiments, wherein said scaffold comprises a polymer.
Embodiment 25. The method according to any of the preceding embodiments, wherein said scaffold comprises polysaccharides and/or polypeptides.
Embodiment 26. The method according to any of the preceding embodiments, wherein said scaffold comprising a polymer selected from the group consisting of polycaprolactone (PCL), and polyurethane and polyacrylate-coated polycaprolactone.
Embodiment 27. The method according to any of the preceding embodiments, wherein said scaffold comprises polycaprolactone (PCL) fibers having a diameter 1 to 10 micrometer(s), such as 4 to 8 micrometer(s), for example about 8 micrometers.
Embodiment 28. The method according to any of the preceding embodiments, wherein said depth of the scaffold is in the range of 0.2 to 2 millimeters, such as 0.5 to 1 millimeter.
Embodiment 29. The method according to any of the preceding embodiments, wherein said scaffold has a surface in the range of 0.5 to 1.5 cm², for example 0.7 to 1.25 cm² , such as 0.7 to 1 cm², for example 0.7 to 0.95 cm², such as 0.7 to 0.85 cm², for example about 0.79 or 0.85 cm².
Embodiment 30. The liver tissue obtained by the method according to embodiments 1 to 29.
Embodiment 31. The liver tissue according to embodiment 30, wherein 90 to 100% of the liver cells are positive for HNF4a and 90 to 100% display CYP1A2 function and 90 to 100% are positive for albumin and 90 to 100% are positive for A1AT secretion.
Embodiment 32. The liver tissue according to embodiment 30 or 31, wherein the liver cells do not or essentially do not express fetal liver protein (AFP). Embodiment 33. The liver tissue obtained by the method according to embodiments 1 to 29 for use as a medicament.
Embodiment 34. The liver tissue obtained by the method according to embodiments 1 to 29 for use in the treatment of acute-on-chronic liver failure (ACLF), acute liver failure, chronic liver failure, preparation for liver implantation, tyrosinemia type I or Alpha-1 antitrypsin deficiency (A1AD or AATD).

### Examples

Pluripotent stem cells (PSCs) were expanded on a blend of laminin extracellular matrices consisting of 521 and 111. When the PSCs reached the correct density, they were exposed to endodermal priming medium containing Activin A and Wnt3a for 72 hours (renewing the medium every 24 hours) and then hepatic progenitor specification medium containing 1% DMSO for 5 days, with medium changes every 48 hours. Stem cell progenitors are removed from their matrix and subsequently used for tissue engineering.

To prime toward the mesoderm, PSCs were exposed to mesodermal priming medium containing CHIR and BMP4 for 72 hours. Following this, cells were incubated in endothelial differentiation medium containing VEGF and forskolin for 48 hours (with a medium change at 24 hours). Endothelial cell populations were subsequently removed from their matrix and enriched using CD144 magnetic beads and then used for liver tissue engineering purposes.

Liver tissue engineering was achieved by cell self-assembly, using microwell technology. PSC derived hepatic progenitors and endothelial cells were removed from their ECMs and then mixed at the ratio 3:1 and formed liver spheres (process here referred as liver sphere aggregation). Stem cell derived liver spheres were removed from the microwells and replated on a polycaprolactone scaffold (Figure 1 shows an example of an empty scaffold). Stem cell derived liver spheres at day 1 (D1) and day 4 (D4) post liver sphere aggregation were used to seed the scaffold structures (Figure 2). The liver tissue phenotype was monitored over 3 weeks post liver tissue attachment to the scaffold. The inventors observed maintenance of mature liver functions (albumin secretion, alpha 1 antitrypsin and cytochrome P450 activity) when fetal liver protein secretion (AFP) decreased to baseline levels (Figure 3). Of particular note, D1 scaffolds demonstrated a significant reduction in an immature marker, AFP. Whilst a mature marker, albumin, was significantly increased at 2 weeks post plating onto the scaffold. By week 3, both scaffolds exhibited baseline levels of AFP secretion, indicating maturity.

The 256 liver spheres (from one micromould) were transferred to polycaprolactone scaffolds of 0.5 mm thickness, 8 µm fiber diameter and 0.95 cm² of surface on a 24-well plate; either 1 day after cell aggregation (here referred as D1 spheres), or 4 days after cell aggregation (here referred as D4 spheres). Each scaffold has a retainer ring above it to prevent it from floating or moving.

Once transferred, medium was routinely changed the following way:
1. Until day 21 post liver sphere aggregation, it was ensured each sample had 1.5 mL of liver sphere medium in each well of their 24-well plate. A volume of 750 µl of consumed medium was aspirated and the same volume of fresh liver sphere medium was added on Mondays and Wednesdays. On Fridays, a volume of 1 mL of consumed medium was aspirated and the same volume of fresh medium was added.
2. From day 21 post liver sphere aggregation onwards (day 21 included), it is ensured that each sample has 2 mL of liver sphere medium in each well of their 24-well plate. A volume of 1 mL of consumed medium was removed and the same volume of fresh liver sphere medium was added on Mondays and Wednesdays. On Fridays, a volume of 1.5 mL of consumed medium was aspirated and the same volume of fresh medium was added.

## Claims

1. A method for preparing liver tissue, said method comprising the step of:
(i) providing endothelial cells, such as human endothelial cells;
(ii) providing hepatic progenitor cells, such as human hepatic progenitor cells;
(iii) preparing a mixture comprising said hepatic progenitor cells and endothelial cells in a culture medium;
(iv) seeding the mixture on a cell culture substrate;
(v) allowing the mixture of cells to propagate and form an aggregation of cells;
(vi) collecting said aggregation of cells after about 1 to 6 days;
(vii) seeding said aggregation of cells on a scaffold and allowing the mixture of cells to propagate and form liver tissue on said scaffold.

2. The method according to claim 1, wherein the aggregation is collected after 1 to 4 days, such as about 1 day or about 4 days.

3. The method according to claim 1 or 2, wherein the ratio of said endothelial cells to hepatic progenitor cells in the mixture is in the range of 1:1 to 1:3.5, for example 1:1 to 1:3, such as 1:1.5, 1:2 or 1:3.3.

4. The method according to any one of the preceding claims, wherein the endothelial cells have been enriched for CD144 positive cells and/or wherein the hepatic progenitor cells have been enriched for AFP and HNF4a positive cells.

5. The method according to any one of the preceding claims, wherein said hepatic progenitor cells have been obtained by seeding pluripotent stem cells on a cell culture substrate comprising a mixture of laminin-521 or a fragment thereof and laminin-111 or a fragment thereof; and culturing the pluripotent stem cells to obtain said hepatic progenitor cells, optionally wherein the weight ratio of laminin-111 to laminin-521 is from about 4:1 to about 1:1.

6. The method according to any one of the preceding claims, wherein said endothelial cells have been obtained by seeding pluripotent stem cells on a cell culture substrate comprising a mixture of laminin-521 or a fragment thereof and laminin-111 or a fragment thereof; and culturing the pluripotent stem cells to obtain said endothelial cells.

7. The method according to claim 6, wherein said endothelial cells have been obtained by seeding pluripotent stem cells on a cell culture substrate comprising a mixture of laminin-521 or a fragment thereof and laminin-111 or a fragment thereof, wherein the concentration by weight of laminin-111 in said cell culture substrate is equal to or higher than the concentration of laminin-521, such as the ratio of laminin-111 to laminin-521 is in the range of 1:1 to 4:1, such as about 3:1.

8. The method according to any of the preceding claims, wherein said scaffold is selected from the group consisting of fibrous scaffold, porous scaffold and hydrogel scaffold.

9. The method according to any of the preceding claims, wherein said scaffold comprises a polymer, such as a scaffold comprising polysaccharides and/or polypeptides.

10. The method according to any of the preceding claims, wherein said scaffold comprising a polymer selected from the group consisting of polycaprolactone (PCL), polyurethane and polyacrylate-coated polycaprolactone.

11. The method according to any of the preceding claims, wherein said scaffold comprises polycaprolactone (PCL) fibers having a diameter 1 to 10 micrometer(s), such as 4 to 8 micrometer(s), for example about 8 micrometers.

12. The method according to any of the preceding claims, wherein said depth of the scaffold is in the range of 0.2 to 2 millimeters, such as 0.5 to 1 millimeter.

13. The method according to any of the preceding claims, wherein said scaffold has a surface in the range of 0.5 to 1.5 cm², for example 0.7 to 1.25 cm² , such as 0.7 to 1 cm², for example 0.7 to 0.95 cm², such as 0.7 to 0.85 cm² for example about 0.79 or 0.95 cm².

14. The liver tissue obtained by the method according any one of claims to 1 to 13.

15. The liver tissue obtained by the method according to any one of claims 1 to 13 for use as a medicament, such as for use in the treatment of acute-on-chronic liver failure (ACLF), acute liver failure, chronic liver failure, preparation for liver implantation, tyrosinemia type I or Alpha-1 antitrypsin deficiency (A1AD or AATD).
